# EUROPEAN PATENT SPECIFICATION

(11) **EP 1 432 562 B1**
(45) Date of publication and mention of the grant of the patent: **23.08.2006**
(21) Application number: 02763088.8
(22) Date of filing: 23.09.2002
(51) Int. Cl.: B29C 41/14, A61F 2/12, A61F 2/52

(54) **APPARATUS AND METHOD FOR MANUFACTURING A SILICONE COVER FOR A BREAST-IMPLANT**
VORRICHTUNG UND VERFAHREN ZUR HERSTELLUNG EINER UMHÜLLUNG AUS SILIKON FÜR EIN BRUSTIMPLANTAT
DISPOSITIF ET PROCEDE PERMETTANT DE FABRIQUER UNE ENVELOPPE EN SILICONE POUR UN IMPLANT MAMMAIRE

(30) Priority: 24.09.2001 NL 1019023
(43) Date of publication of application: 30.06.2004
(73) Proprietor: Mentor Medical Systems B.v., 2333 CL Leiden (NL)
(72) Inventor: SMIT, Jan, Johan, Hendrik, NL-2403 HR Alphen A/D Rijn (NL)
(74) Representative: Van Breda, Jacobus
(86) International application number: PCT/NL2002/000611
(87) International publication number: WO 2003/026866

(56) References cited:
- EP-A- 0 315 814
- EP-A- 0 416 846
- BE-A- 828 143

## Description

The invention relates to an apparatus and method for manufacturing a silicone cover for a breast implant according to the preamble of claims 1 and 6 respectively. Such an apparatus and method are known from EP-A-0 416 846.

Silicone covers are commonly produced by dipping the mould that is being used, for example, four times in a silicone solution. The silicone solution used is a methyl solution alternating with a phenyl solution. The purpose of repeated dipping is to provide the silicone cover with the desired thickness.

The phenyl solution is applied as second layer and serves as boundary layer, in order to prevent the so-called "bleeding" or leaking.

Mixing the silicone solution with xylene provides the desired viscosity. When applying four dips, a methylphenyl-methyl-methyl layer is obtained.

EP-A-0 416 846 teaches a method for manufacturing a silicone cover for a breast implant by repeatedly dipping a mould for this cover in a plastic solution such that each time an edge of the curved upper side is immerged first. After the mould has been repeatedly dipped, at least one curing treatment takes place.

It is the object of the invention to improve the quality of the covers.

Accordingly, the invention provides an apparatus and a method according to claims 1 and 6 respectively

Applicants have found that this measure solves the problem that a weak spot is left in the manufactured silicone cover caused by the air inclusions in the plastic layer.

With the invention such air inclusions are effectively avoided.

Between the dips, there is in general one evaporation phase in which solvent can evaporate. After the last dip this evaporation may preferably take place at elevated temperature.

A suitable embodiment of the apparatus is characterized in that the arm has a pivoting point located above the plastic solution, about which pivoting point the arm is adjustable over at least 90°.

In order to provide an effective throughput capacity, the apparatus is further characterized in that, viewed in the direction of processing, the same is provided with an evaporating oven placed after the dipping station, for evaporating an excess of solvent from the plastic solution present on the mould.

It is further useful for a curing oven to be provided succeeding the evaporating oven.

It has further been shown to be desirable for the evaporating oven to be equipped with an inlet and outlet for air, with the inlet in relation to the mould being located in the top of the evaporating oven and the outlet being located at a level with a dipping tank for the mould, provided in the dipping station.

This measure has been shown to be especially important for the effective evaporation of the excess of xylene originating from the plastic solution, without any weak spots being left behind in the final silicone covers manufactured with the apparatus and method in accordance with the invention. This also avoids hazardous fume concentrations in the solvent.

The invention will now be further elucidated with reference to the drawing, which
- in Fig. 1 shows the apparatus according to the invention in a cross-sectional side view;
- in Fig. 2 shows a cross-section of the apparatus according to the invention at the position of the dipping station.

Identical reference numbers used in the figures refer to similar parts.

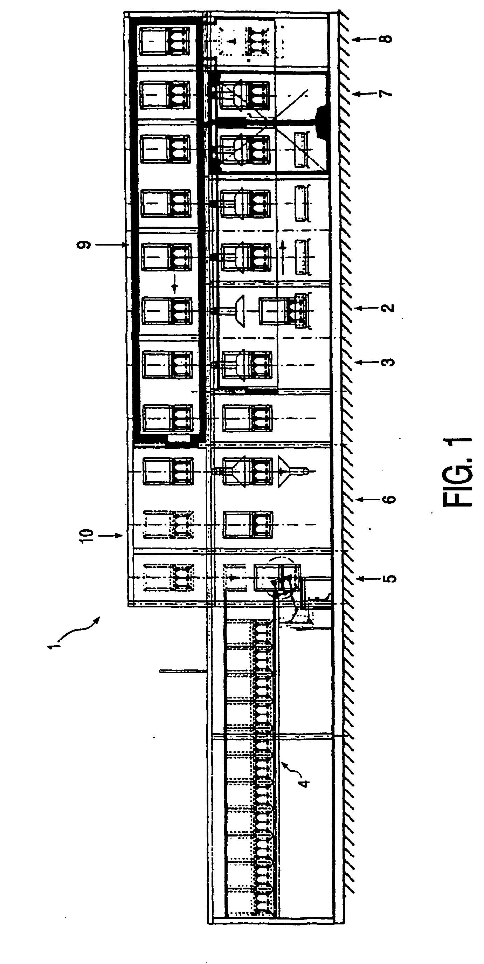

Fig. 1 shows the apparatus 1, which serves for the substantially automatic manufacture of a silicone cover for a breast implant. A part of this apparatus 1 is a dipping station 2, wherein a mould is repeatedly dipped into a plastic solution in order to form said cover. It is also possible to provide several, for example, three dipping stations in succession for the repeated dipping of the mould.

Preceding the dipping station (viewed in the installation's direction of operation) there is a preheating oven 3 in which the mould or moulds are preheated. In the case illustrated, the moulds are placed in threefold on a common mandrel that is driven by a conveyer 4. Modifications to this are of course possible.

At the input station 5 the moulds are introduced into the actual apparatus 1 after which the moulds are cleaned in the cleaning station 6. After this cleaning station 6 the moulds are preheated in the preheating oven 3, in the dipping station 2 (or in several dipping stations) the moulds are repeatedly dipped into a plastic solution and the moulds are conveyed further to an evaporating oven 7 for the evaporation of xylene from the plastic solution with which the moulds are coated.

From this evaporating oven 7 the moulds are transported upward by means of a lift 8 to a curing oven 9 where the plastic coatings provided on the moulds are cured. The curing oven 9 is succeeded by a cooling section, and transport to the exhaust area as indicated by reference number 10.

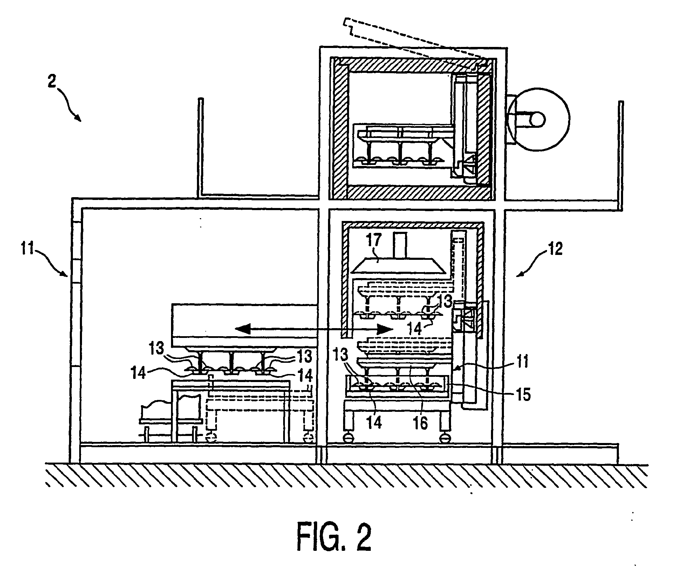

For a further elucidation of the dipping as such, reference is made to Fig. 2.

Fig. 2 shows the dipping station 2 in a cross-sectional view. This dipping station 2 comprises a dipping section 11 and an oven section 12. Both in the dipping section 11 and in the oven section 12, moulds 13 are shown that are placed upside down on an arm 14 allowing vertical transport, in order bring them into a tank 15 filled with a plastic solution, as shown in the dipping section 11. After each such immersion, the moulds 13 are conveyed to an oven section 12 for the exhaustion of xylene that is present in the plastic solution in the mould 15, in order to adjust the desired viscosity.

An essential aspect of the invention is that during the process the arm 14 is operated such that at least prior to the moulds 13 being totally immersed in the bath of plastic in the tank 15, an upper end of the mould 13 is contacted with this plastic solution by adjusting the arm 14 such that it forms an angle with the vertical in a manner that allows the upper end of the mould 13 to be dipped into the plastic solution before the entire mould 13 disappears in this solution. To this end the arm 14 is provided with a pivoting point located above the plastic solution at the height of a connecting shaft 16, to which in the case illustrated further arms 14 with moulds 13 are coupled, and at which pivoting point the arms 14 can be adjusted over at least 90°.

The evaporating oven 7 has an inlet 17 and an outlet 18 for air wherein, with respect to the mould 13, the inlet 17 is located in the top of the evaporating oven 7, and wherein the outlet 18 is located at the height of a dipping tank for the mould that is provided in the dipping station 2. This is completely obvious to the person skilled in the art and is therefore not shown in the figure.

The above specification must be understood to be a non-limiting exemplary embodiment of the apparatus and method according to the invention. Numerous variations and modifications may be applied to this apparatus and method, all falling within the scope of the invention. The protection to be conferred on the invention is therefore determined by the appended claims only.

## Claims

1. An apparatus (1) for manufacturing a silicone cover for a breast implant, having a dipping station (2) for repeatedly immersing at least one mould of said cover in a plastic solution, wherein the mould (13) at its lower side is placed on an adjustable arm (14), such that the upper side of the mould (13) stays free of obstructions, **characterized in that** during operation the arm (14) can be adjusted in order to position the mould (13) so as to first allow an upper end of the mould (13) to come in contact with the plastic solution prior to immersing the entire mould (13) in said solution.

2. An apparatus according to claim 1, **characterized in that** the arm (14) has a pivoting point located above the plastic solution, about which pivoting point the arm (14) is adjustable over at least 90°.

3. An apparatus according to one of the preceding claims, **characterized in that** viewed in the direction of processing, the same is provided with an evaporating oven (7) placed after the dipping station (2), for evaporating an excess of solvent from the plastic solution present on the mould (13).

4. An apparatus according to claim 3, **characterized in that** a curing oven (9) is provided succeeding the evaporating oven (7).

5. An apparatus according to claim 3 or 4, **characterized in that** the evaporating oven (7) is equipped with an inlet (17) and outlet (18) for air, with the inlet (17) in relation to the mould (13) being located in the top of the evaporating oven (7) and the outlet (18) being located at the height of a dipping tank for the mould, provided in the dipping station (2).

6. A method for manufacturing a silicone cover for a breast implant wherein a mould (13) for this cover is repeatedly immersed in a plastic solution, and that after repeated immersing of the mould (13), at least one curing treatment takes place, **characterized in that** prior to immersing the entire mould in said solution, an upper end of the mould is first allowed to come in contact with the solution,

7. A method according to claim 6, **characterized in that** after the last immersing operation, solvent is evaporated at an elevated temperature.

## Patentansprüche

1. Eine Vorrichtung (1) zum Herstellen einer Silikonumhüllung für ein Brustimplantat, die eine Tauchstation (2) zum wiederholten Eintauchen zumindest einer Form der Umhüllung in einer Kunststofflösung aufweist, wobei die Form (13) an ihrer unteren Seite an einem einstellbaren Arm (14) platziert ist, derart, dass die obere Seite der Form (13) frei von Hindernissen bleibt, **dadurch gekennzeichnet, dass** während eines Betriebs der Arm (14) eingestellt werden kann, um die Form (13) zu positionieren, um zuerst zu ermöglichen, dass ein oberes Ende der Form (13) in Kontakt mit der Kunststofflösung kommt, bevor die ganze Form (13) in die Lösung eingetaucht wird.

2. Eine Vorrichtung gemäß Anspruch 1, **dadurch gekennzeichnet, dass** der Arm (14) einen Schwenkpunkt aufweist, der über der Kunststofflösung angeordnet ist, wobei der Arm (14) um diesen Schwenkpunkt über zumindest 90° einstellbar ist.

3. Eine Vorrichtung gemäß einem der vorhergehenden Ansprüche, **dadurch gekennzeichnet, dass**, in der Verarbeitungsrichtung betrachtet, dieselbe mit einem Verdampfungsofen (7) ausgestattet ist, der nach der Tauchstation (2) platziert ist, zum Verdampfen von überschüssigem Lösungsmittel von der Kunststofflösung, die auf der Form (13) vorhanden ist.

4. Eine Vorrichtung gemäß Anspruch 3, **dadurch gekennzeichnet, dass** ein Aushärtungsofen (9) nach dem Verdampfungsofen (7) bereitgestellt ist.

5. Eine Vorrichtung gemäß Anspruch 3 oder 4, **dadurch gekennzeichnet, dass** der Verdampfungsofen (7) mit einem Einlass (17) und einem Auslass (18) für Luft ausgestattet ist, wobei der Einlass (17) bezüglich der Form (13) in dem oberen Ende des Verdampfungsofens (7) angeordnet ist und der Auslass (18) auf der Höhe eines Tauchtanks für die Form angeordnet ist, der in der Tauchstation (2) bereitgestellt ist.

6. Ein Verfahren zum Herstellen einer Silikonumhüllung für ein Brustimplantat, wobei eine Form (13) für diese Umhüllung wiederholt in eine Kunststofflösung eingetaucht wird, und dass nach einem wiederholten Eintauchen der Form (13) zumindest eine Aushärtungsbehandlung stattfindet, **dadurch gekennzeichnet, dass** vor einem Eintauchen der ganzen Form in die Lösung zuerst ermöglicht wird, dass ein oberes Ende der Form in Kontakt mit der Lösung kommt.

7. Ein Verfahren gemäß Anspruch 6, **dadurch gekennzeichnet, dass** nach der letzten Eintauchoperation ein Lösungsmittel bei einer erhöhten Temperatur verdampft wird.

## Revendications

1. Appareil (1) pour fabriquer une enveloppe en silicone pour un implant mammaire, ayant un poste de trempage (2) pour immerger de manière répétée au moins un moule de ladite enveloppe dans une solution de matériau plastique, dans lequel le moule (13), sur son côté inférieur, est placé sur un bras ajustable (14) de sorte que le côté supérieur du moule (13) reste dégagé de toutes obstructions, **caractérisé en ce que**, en cours de fonctionnement, le bras (14) peut être ajusté pour positionner le moule (13) de manière à permettre d'abord à une extrémité supérieure du moule (13) de venir en contact avec la solution de matériau plastique avant d'immerger tout le moule (13) dans ladite solution.

2. Appareil selon la revendication 1, **caractérisé en ce que** le bras (14) a un point pivot placé au-dessus de la solution de matériau plastique, autour duquel point pivot le bras (14) peut être ajusté sur au moins 90°.

3. Appareil selon l'une quelconque des revendications précédentes, **caractérisé en ce que**, observé dans le sens du traitement, celui-ci est muni d'un four d'évaporation (7) placé après le poste de trempage (2) pour évaporer un excès de solvant de la solution de matériau plastique présent sur le moule (13).

4. Appareil selon la revendication 3, **caractérisé en ce qu'**un four de durcissement (9) est aménagé à la suite du four d'évaporation (7).

5. Appareil selon la revendication 3 ou 4, **caractérisé en ce que** le four d'évaporation (7) est équipé d'une entrée (17) et d'une sortie (18) pour l'air, l'entrée (17) étant, par rapport au moule (13), placée dans la partie supérieure du four d'évaporation (7) et la sortie (18) étant située à la hauteur d'un réservoir de trempage pour le moule aménagé dans le poste de trempage (2).

6. Procédé pour fabriquer une enveloppe en silicone pour un implant mammaire, dans lequel un moule (13) destiné à cette enveloppe est immergé de manière répétée dans une solution de matériau plastique et, après immersion répétée du moule (13), on effectue au moins un traitement de durcissement, **caractérisé en ce que**, avant d'immerger tout le moule dans ladite solution, on laisse d'abord une extrémité supérieure du moule venir en contact avec la solution.

7. Procédé selon la revendication 6, **caractérisé en ce que**, après la dernière opération d'immersion, on laisse le solvant s'évaporer à température élevée.
